# EUROPEAN PATENT SPECIFICATION

(11) **EP 4 238 537 B1**
(45) Date of publication and mention of the grant of the patent: **06.05.2026**
(21) Application number: 23159342.7
(22) Date of filing: 01.03.2023
(51) Int. Cl.: A61F 2/16

(54) **INTRAOCULAR LENS INSERTION INSTRUMENT**
INSTRUMENT ZUM EINSETZEN EINER INTRAOKULARLINSE
INSTRUMENT D'INSERTION DE LENTILLE INTRAOCULAIRE

(30) Priority: 02.03.2022 JP 2022031684
(43) Date of publication of application: 06.09.2023
(73) Proprietor: Nidek Co., Ltd., Gamagori-shi, Aichi, 443-0038 (JP)
(72) Inventor: INOUE, Youta, Gamagori-shi, 443-0038 (JP); NAGASAKA, Shinji, Gamagori-shi, 443-0038 (JP); HISHIDA, Takahiro, Gamagori-shi, 443-0038 (JP); KAMIYA, Tomoaki, Gamagori-shi, 443-0038 (JP)
(74) Representative: TBK

(56) References cited:
- EP-A1- 2 641 568
- WO-A1-2012/086797
- WO-A2-98/37830
- CABEZA-GIL I ET AL: "Experimental evaluation of the injection force exerted in intraocular lens delivery with syringe-type injectors", JOURNAL OF THE MECHANICAL BEHAVIOR OF BIOMEDICAL MATERIALS, ELSEVIER, AMSTERDAM, NL, vol. 124, 30 August 2021 (2021-08-30), XP086833390, ISSN: 1751-6161, [retrieved on 20210830], DOI: 10.1016/J.JMBBM.2021.104793

## Description

### BACKGROUND

### 1. Technical Field

The present disclosure relates to an intraocular lens insertion instrument.

### 2. Related Art

As one of methods of cataract surgery, a method for inserting a soft foldable intraocular lens into the eye instead of the crystalline lens is generally used. Moreover, an intraocular lens may be inserted forward of the crystalline lens to correct the refractive power of the eye. An intraocular lens insertion instrument called an injector may be used to insert an intraocular lens into the eye.

Such an injector is disclosed in, for example, Japanese Patent JP 5 658 992 B2. In a form described in Japanese Patent JP 5 658 992 B2, an intraocular lens is pushed, by means of a pushing shaft, in a nozzle having a hollow passage that allows passage of the intraocular lens therethrough and that gradually reduces in area toward a distal end thereof. Consequently, the intraocular lens is folded into a small size, and ejected to the outside from the distal end of the nozzle. An injector such as described in Japanese Patent JP 5 658 992 B2 is inserted into the eye through the wound of an incision made in the cornea. In recent years, the trend toward micro-incision surgery is accelerating in cataract surgery. In other words, it is desired to make an insertion portion, which is the nozzle end of the injector, narrow in response to a demand for micro-incision surgery. WO 2012 / 086 797 A1 discloses an intraocular lens insertion instrument having the features in the preamble of claim 1. EP 2 641 568 A1 and WO 98 / 37 830 A2 disclose further prior art.

### SUMMARY

An intraocular lens insertion instrument including: a tubular main body portion where an intraocular lens is placed; a rod-like pushing member inserted into the main body portion and configured to press the intraocular lens from a back toward a front of the main body portion in an axial direction, thereby push the intraocular lens in a folded state from a distal end of the main body portion to the outside, and then insert the intraocular lens into an eye; and a cylindrical insertion portion placed in the distal end of the main body portion to be inserted into the eye, in which the insertion portion includes a stress concentrated area where stress to be generated upon the folded intraocular lens passing through the insertion portion is concentrated, and the insertion portion includes a thick-walled structure in at least a part thereof, in which a thickness of the stress concentrated area of the insertion portion is set to be relatively greater than a thickness of an area of the insertion portion outside the stress concentrated area as the insertion portion is viewed in a direction of a central axis thereof. Further, in the intraocular lens insertion instrument, the insertion portion includes, in a distal end thereof, an inclined opening end surface inclined relative to a virtual plane orthogonal to the central axis of the insertion portion. The insertion portion includes: an opening tip portion located at an extreme tip of the inclined opening end surface; and a slit cut backward in the axial direction from an end surface located at a backmost end of the inclined opening end surface. The thick-walled structure is formed on at least one of a side surface of a portion, which serves as the opening tip portion, of the insertion portion, and a side surface of a portion, which serves as the slit, of the insertion portion.

### BRIEF DESCRIPTION OF THE DRAWINGS

Fig. 1 is an entire perspective view illustrating an intraocular lens insertion instrument;
Fig. 2 is a perspective view illustrating a plunger;
Fig. 3 is a plan view of an intraocular lens;
Fig. 4 is a right side view of the intraocular lens;
Fig. 5 is a side view illustrating a placement portion and a nozzle of the intraocular lens insertion instrument;
Fig. 6 is a plan view illustrating the placement portion and the nozzle of the intraocular lens insertion instrument;
Fig. 7 is an enlarged view illustrating an enlarged insertion portion of the nozzle of the intraocular lens insertion instrument;
Fig. 8 is a cross-sectional view taken along line VIII-VIII of Fig. 7;
Fig. 9 is a cross-sectional view taken along line IX-IX of Fig. 7;
Fig. 10 is across-sectional view taken along line X-X of Fig. 7;
Fig. 11 is a schematic explanatory diagram illustrating a state where the intraocular lens is placed in the placement portion;
Fig. 12 is a schematic explanatory diagram illustrating a state where a pushing member has started pushing the intraocular lens;
Fig. 13 is a schematic explanatory diagram illustrating a state where the intraocular lens has moved toward the nozzle;
Fig. 14 is a schematic explanatory diagram illustrating a state where the intraocular lens has started making an entry into the nozzle;
Fig. 15 is a schematic explanatory diagram illustrating a state where the intraocular lens deforms in the nozzle;
Fig. 16 is a schematic explanatory diagram illustrating a state where the intraocular lens has deformed further in the nozzle;
Fig. 17 is a schematic explanatory diagram illustrating a state where the intraocular lens is folded in the nozzle; and
Fig. 18 is a partially enlarged view illustrating the folded intraocular lens in the insertion portion.

### DETAILED DESCRIPTION

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

The size of an intraocular lens used as a crystalline lens substitute is generally adjusted to the size of the eye. Hence, the size of the intraocular lens hardly changes significantly. Therefore, if an insertion portion, which is a nozzle end portion, is made narrow in response to the demand for micro-incision surgery, a load on the insertion portion to be applied when the intraocular lens is ejected tends to increase. Moreover, the hardness of the intraocular lens varies. However, the intraocular lens generally exhibits the tendency to increase hardness with decreasing temperature due to the properties of polymers.

One object of the present disclosure is to provide an intraocular lens insertion instrument that improves resistance to a load on an insertion portion in a nozzle end to be applied when an intraocular lens is ejected.

The above object is solved by an intraocular lens insertion instrument according to claim 1.

According to the intraocular lens insertion instrument, it is possible to provide an intraocular lens insertion instrument that can improve resistance to a load on an insertion portion in a nozzle end to be applied when an intraocular lens is ejected.

### <Overview>

The intraocular lens insertion instrument illustrated by example in the present disclosure includes: a tubular main body portion where an intraocular lens is placed; a rod-like pushing member inserted into the main body portion and configured to press the intraocular lens from a back toward a front of the main body portion in an axial direction, thereby push the intraocular lens in a folded state from a distal end of the main body portion to the outside, and then insert the intraocular lens into an eye; and a cylindrical insertion portion placed in the distal end of the main body portion to be inserted into the eye. The insertion portion includes a stress concentrated area where stress (for example, stress on the intraocular lens, stress on a back support portion, and stress on a front support portion) to be generated upon the folded intraocular lens passing through the insertion portion is concentrated. The insertion portion includes a thick-walled structure in at least a part thereof. In the thick-walled structure, a thickness of the stress concentrated area of the insertion portion is set to be relatively greater than a thickness of an area of the insertion portion outside the stress concentrated area as the insertion portion is viewed in a direction of a central axis thereof. Consequently, it is possible to provide an intraocular lens insertion instrument that improves resistance to a load on the insertion portion in a nozzle end to be applied when the intraocular lens is ejected.

Moreover, in the above intraocular lens insertion instrument, the stress concentrated area of the insertion portion may be an area where the stress is concentrated in response to a repulsive force generated in a direction where the folded intraocular lens recovers an original shape thereof (a force in a direction where the intraocular lens recovers an original (unfolded) attitude thereof). Consequently, it is possible to improve resistance to a load on the area of the insertion portion in the nozzle end where the stress is concentrated in response to the repulsive force generated in the direction where the folded intraocular lens recovers the original shape (the force in the direction where the intraocular lens recovers the original (unfolded) attitude.

Further, in the intraocular lens insertion instrument, there may be provided a tapered portion which is continuous with a back of the insertion portion in the axial direction, the tapered portion having a hollow passage shape where a passage through which the intraocular lens passes gradually reduces in area toward a distal end thereof. The intraocular lens may include: a disc-shaped optical portion; a front support portion extending outward in a radial direction from an outer edge portion of the optical portion, the front support portion being located on a front side in the axial direction, which is a side closer to the insertion portion, in the tapered portion; and a back support portion extending outward in the radial direction from the outer edge portion of the optical portion, the back support portion being located on a back side in the axial direction, which is opposite to the insertion portion, in the tapered portion. As the intraocular lens is pushed forward in the axial direction by the pushing member in the tapered portion, the intraocular lens may become folded in such a manner that, adjusting to the passage shape of the tapered portion, a distal end portion of the front support portion and a distal end portion of the back support portion may be folded onto the optical portion in a direction of moving closer to each other and the optical portion may wrap the front support portion and the back support portion, and the stress concentrated area may be an area where the stress is concentrated in response to a repulsive force generated in a direction where the back support portion of the folded intraocular lens recovers the original shape.

As described above, the stress concentrated area is the area where the stress is concentrated in response to the repulsive force generated in the direction where the back support portion of the folded intraocular lens recovers an original shape thereof. The vicinity of a basal portion of the back support portion of the intraocular lens that is pushed forward in the axial direction by the pushing member tends to be pressed against an inner surface of the insertion portion as the back support portion is folded. Hence, the repulsive force generated in the recovering direction is likely to increase in the back support portion. Moreover, due to the repulsive force generated in the direction where the back support portion recovers the original shape, a pushing force acts on a distal end of the pushing member in an opposite direction toward no provided basal portion of the back support portion. Hence, a load is likely to be applied to an inner surface on the opposite side of the insertion portion where the back support portion is not provided. Therefore, if the thick-walled structure is provided to the stress concentrated area, it is possible to efficiently improve resistance to a load on the insertion portion in the nozzle end to be applied when the intraocular lens is ejected.

Further, in the intraocular lens insertion instrument, the insertion portion includes, in a distal end thereof, an inclined opening end surface inclined relative to a virtual plane orthogonal to the central axis of the insertion portion. The insertion portion includes: an opening tip portion located at an extreme tip of the inclined opening end surface; and a slit cut backward in the axial direction from an end surface located at a backmost end of the inclined opening end surface. The thick-walled structure is formed on at least one of a side surface of a portion, which serves as the opening tip portion, of the insertion portion, and a side surface of a portion, which serves as the slit, of the insertion portion.

In this configuration, the stress concentrated area is on the above-mentioned side surface of the portion, which serves as the opening tip portion, of the insertion portion or side surface of the portion, which serves as the slit, of the insertion portion. Hence, the thick-walled structure is provided to at least one of the areas; therefore, it is possible to selectively improve resistance to a load on the insertion portion in the nozzle end to be applied when the intraocular lens is ejected.

Moreover, the thick-walled structure of the above intraocular lens insertion instrument may be formed on each of the side surface of the portion, which serves as the opening tip portion, of the insertion portion, and the side surface of the portion, which serves as the slit, of the insertion portion. Consequently, it is possible to further improve resistance to a load on the insertion portion in the nozzle end to be applied when the intraocular lens is ejected, on both of the side surface of the portion, which serves as the opening tip portion, of the insertion portion and the side surface of the portion, which serves as the slit, of the insertion portion.

Moreover, the thick-walled structure of the above intraocular lens insertion instrument may be formed in such a manner that the side surface of the portion, which serves as the opening tip portion, of the insertion portion is different in thickness from the side surface of the portion, which serves as the slit, of the insertion portion. Consequently, the area of the side surface of the portion, which serves as the opening tip portion, of the insertion portion, or the side surface of the portion, which serves as the slit, of the insertion portion, to whichever a heavier load is applied, is made thicker; therefore, it is possible to excellently improve resistance to the load on the insertion portion.

Moreover, the thick-walled structure of the above intraocular lens insertion instrument may be formed in such a manner that the side surface of the portion, which serves as the opening tip portion, of the insertion portion is equal in thickness to the side surface of the portion, which serves as the slit, of the insertion portion. In other words, the side surface of the portion, which serves as the opening tip portion, of the insertion portion, and the side surface of the portion, which serves as the slit, of the insertion portion are formed in such a manner as to have the same thickness; therefore, it is possible to prevent the areas where the stress is concentrated from being distributed unevenly to one side.

### <Embodiment>

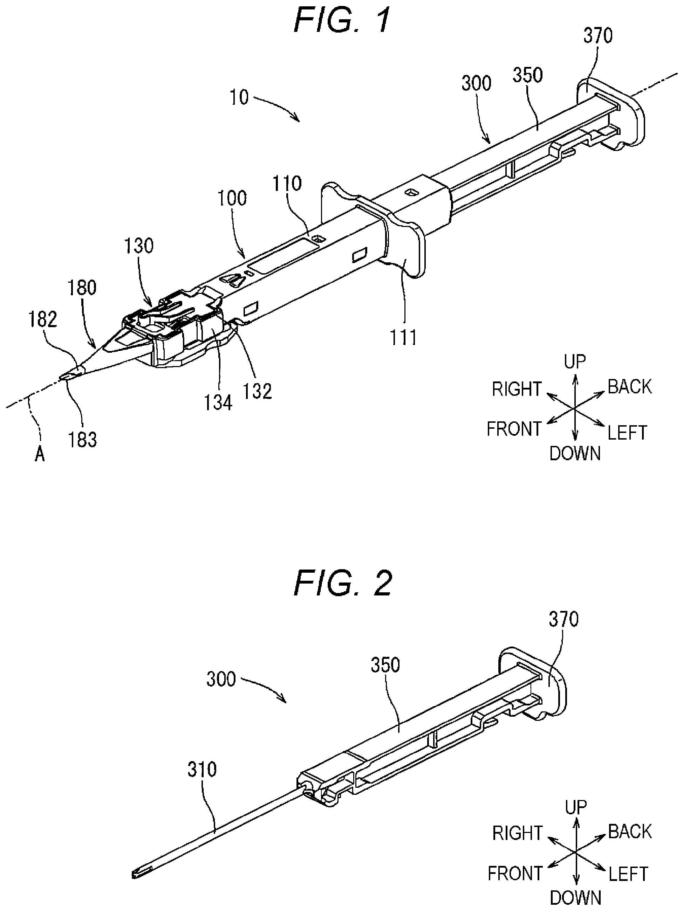

One of typical embodiments of the present disclosure is described hereinafter with reference to Figs. 1 to 18.

Directions indicated in the drawings in the following description are determined as follows: In other words, a direction pointing to a distal end, which is closer to a nozzle 180, of a main body portion 100 in an intraocular lens insertion instrument 10 (to the bottom left of the page in Fig. 1) is assumed to be forward in the intraocular lens insertion instrument 10. Moreover, a direction pointing to a pressing portion 370 of a plunger 300 (to the top right of the page in Fig. 1) is assumed to be backward in the intraocular lens insertion instrument 10. Moreover, the top of the page in Fig. 1 is assumed to be up in the intraocular lens insertion instrument 10. The bottom of the page in Fig. 1 is assumed to be down in the intraocular lens insertion instrument 10. Furthermore, the bottom right of the page in Fig. 1 is assumed to be left in the intraocular lens insertion instrument 10. The top left of the page in Fig. 1 is assumed to be right in the intraocular lens insertion instrument 10.

### <Entire Configuration of Intraocular Lens Insertion Instrument 10>

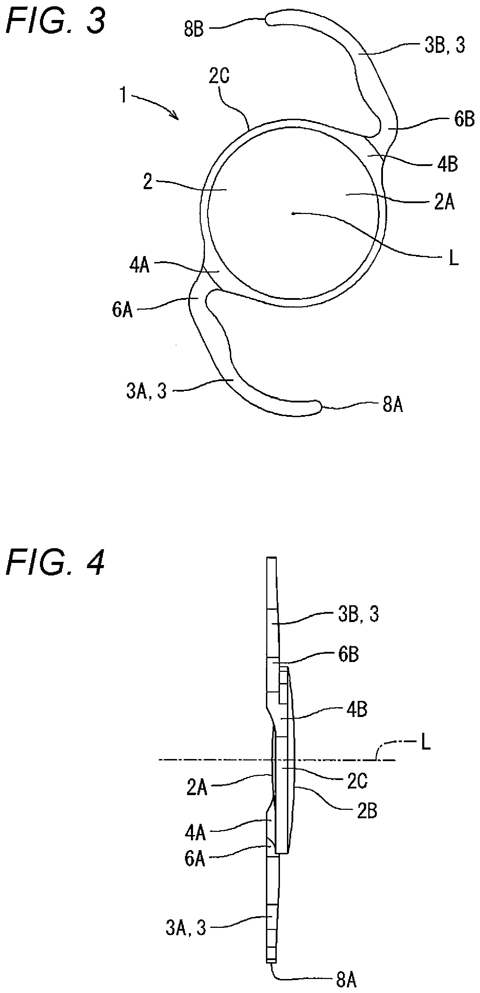

The entire configuration of the intraocular lens insertion instrument 10 of the embodiment is described with reference to Fig. 1. The intraocular lens insertion instrument 10 is used to insert a deformable intraocular lens 1 (refer to Figs. 3 and 4 and details thereof are described below) into the eye. The intraocular lens insertion instrument 10 includes the main body portion 100 and the plunger 300. The main body portion 100 has an approximately tubular shape. The intraocular lens 1 is inserted into the eye through a passage inside the main body portion 100. The plunger 300 is a rod-like member, and can move in a front-and-back direction (a direction along a extrusion axis A) through the passage inside the main body portion 100. The plunger 300 moves forward along the extrusion axis (the axis of the passage) A to push out the intraocular lens 1 loaded into the main body portion 100.

The main body portion 100 and the plunger 300 of the intraocular lens insertion instrument 10 of the embodiment are formed by, for example, injection molding that uses a resin material (for example, polypropylene). The intraocular lens insertion instrument 10 may be formed by, for example, cutting performed by shaving resin. The intraocular lens insertion instrument 10 may be of a cartridge type that includes the replaceable nozzle 180. The intraocular lens insertion instrument 10 is formed in such a manner as to include the resin material (for example, is formed of substantially only the resin material) to allow a user to easily dispose of the used intraocular lens insertion instrument 10. The intraocular lens insertion instrument 10 in a form illustrated by example in the embodiment is of what is called a preloaded type, and is shipped preloaded with the intraocular lens 1.

In the embodiment, a lubrication coating process is performed on an inner wall of the main body portion 100 to smoothly insert the soft intraocular lens 1 with adhesion properties into the eye. Moreover, the intraocular lens insertion instrument 10 of the embodiment is formed colorless and transparent or colorless and translucent. Therefore, the user can easily view, for example, the deformed state of the intraocular lens 1 loaded into the intraocular lens insertion instrument 10 from outside the intraocular lens insertion instrument 10.

### <Main Body Portion 100>

The tubular main body portion 100 where the intraocular lens 1 is placed is described with reference to Fig. 1. The main body portion 100 includes a main body tubular portion 110, a placement portion 130, and the nozzle 180 from the back to the front.

The main body tubular portion 110 is formed into a tubular shape extending in the front-and-back direction. The main body tubular portion 110 is located on the back end side of the main body portion 100. A flange portion 111 jutting out from an outer peripheral surface of the main body tubular portion 110 is provided at a position on the back end side of the main body tubular portion 110 in a longitudinal direction. The user holds the intraocular lens insertion instrument 10 with his/her fingers on the flange portion 111 during use.

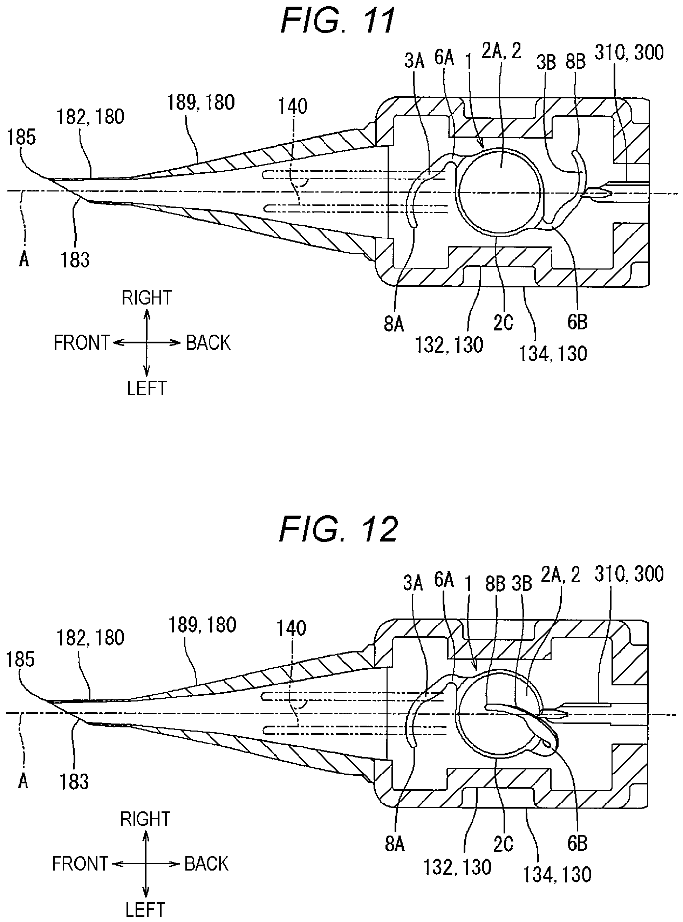

The placement portion 130 is connected to the front end of the main body tubular portion 110. The placement portion 130 includes a placement portion main body 134 and a top portion 132. The placement portion 130 is formed in such a manner that an interior thereof is tubular. The placement portion main body 134 is a box-shaped member that opens at the top. The intraocular lens 1 is placed (loaded) in the placement portion main body 134 of the placement portion 130 before being pushed by the plunger 300 (refer to Figs. 11 and 12).

The top portion 132 is a lid member that is placed astride the nozzle 180 and the placement portion main body 134 and covers an opening at the top of the nozzle 180 and the opening at the top of the placement portion main body 134. The top portion 132 may be formed by, for example, injection molding that uses a resin material (for example, polypropylene), or cutting performed by shaving resin. The top portion 132 has a flat plate shape, and is formed in such a manner as to cover the openings of the nozzle 180 and the placement portion main body 134. The top portion 132 is provided with a grooved guide portion 140 that guides the plunger 300 along the pushing direction (the extrusion axis A).

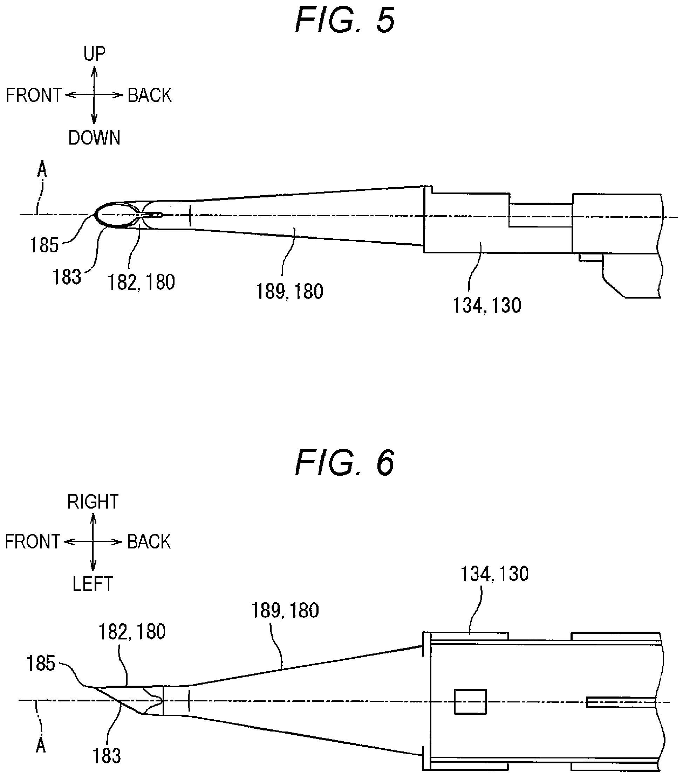

The nozzle 180 is connected to the front end of the placement portion 130 as illustrated in Figs. 5, 6, and 11 to 17. The nozzle 180 includes an insertion portion 182 and a tapered portion 189.

In the tapered portion 189, an internal passage reduces in area toward the front to deform the intraocular lens 1 into a reduced size in the course of pressing the intraocular lens 1 forward. In other words, the tapered portion 189 has a hollow passage shape (bore shape) where the passage through which the intraocular lens 1 passes becomes gradually thinner at the distal end. In other words, the tapered portion 189 is continuous with the back of the insertion portion 182 in the axial direction, and has a hollow passage shape where the passage through which the intraocular lens 1 passes gradually reduces in area toward the distal end.

As illustrated in Figs. 5 to 7, the insertion portion 182 is provided in a connected manner in the front end of the nozzle 180. In other words, the insertion portion 182 is placed in the distal end of the main body portion 100. The insertion portion 182 is a portion that is inserted into (put or placed within) the eye. The insertion portion 182 is provided in a cylindrical form. The insertion portion 182 has an approximately circular cross section. The central axis of the insertion portion 182 and the extrusion axis A agree with each other. An inclined opening end surface 183 (a bevel) that is open to deliver the intraocular lens 1 forward through the internal passage is formed at the distal end of the insertion portion 182. The inclined opening end surface 183 is inclined relative to a virtual plane orthogonal to the central axis of the insertion portion (the extrusion axis A), and is provided in a cut form obtained by cutting the distal end obliquely. The passage inside the main body portion 100 penetrates from the back end of the main body tubular portion 110 to the inclined opening end surface 183 at the front end of the nozzle 180. The insertion portion 182 includes an opening tip portion 185 located at the extreme tip of the inclined opening end surface 183. Moreover, the insertion portion 182 includes a slit 187 cut backward in the axial direction from an end surface located at the backmost end of the inclined opening end surface 183.

### <Plunger 300>

The schematic configuration of the plunger 300 is described with reference to Fig. 2. The plunger 300 of the embodiment includes a pushing member 310, a shaft base 350, and the pressing portion 370.

The pressing portion 370 is formed at the back end of the plunger 300. The pressing portion 370 is a plate-shaped member that extends in a direction orthogonal to the extrusion axis A (refer to Fig. 1). The pressing portion 370 is a portion that the user's finger touches when the user pushes the plunger 300 forward.

The shaft base 350 is a rod-like member that extends forward from the front end of the pressing portion 370. In the embodiment, the shaft base 350 is formed in such a manner as to have an approximately H-shaped cross section that is orthogonal to the extrusion axis A. The shaft base 350 is inserted into the main body tubular portion 110 that has an approximately rectangular cross section that is orthogonal to the extrusion axis A. Consequently, the rotation of the plunger 300 in a circumferential direction of the extrusion axis A relative to the main body portion 100 is prevented. When the plunger 300 moves forward and reaches a position where the insertion of the intraocular lens 1 into the eye is completed, an inclined surface on the bottom at the front end of the shaft base 350 comes into contact with an inclined surface formed in a predetermined place on the main body portion 100, and then stops. As a result, the front end of the plunger 300 is prevented from protruding excessively from the inclined opening end surface 183 (refer to Fig. 1).

The pushing member 310 is a rod-like member inserted into the main body portion 100, and extends forward from the front end of the shaft base 350 along the axial direction of the extrusion axis A. The pushing member 310 is formed in such a manner as to have an approximately circular cross section that is orthogonal to the extrusion axis A. Moreover, the pushing member 310 has a size that allows passage of the pushing member 310 through the inclined opening end surface 183 of the main body portion 100. The pushing member 310 moves forward along the extrusion axis A through the passage of the main body portion 100 to tuck the intraocular lens 1 and also to deliver the intraocular lens 1 into the eye from the inclined opening end surface 183.

In other words, the pushing member 310 is a member that presses the intraocular lens 1 from the back toward the front of the main body portion 100 in the axial direction, thereby pushes the intraocular lens 1 in a folded state thereof from the distal end of the main body portion 100 to the outside, and then inserts the intraocular lens 1 into the eye.

### <Intraocular Lens>

An example of the intraocular lens 1 that is inserted into the eye by means of the intraocular lens insertion instrument 10 is described with reference to Figs. 3 and 4. The intraocular lens 1 includes an optical portion 2 and support portions 3. The intraocular lens 1 of the embodiment is what is called a one-piece intraocular lens where the optical portion 2 and the support portions 3 are integrally molded. In the intraocular lens 1 used in the embodiment, the optical portion 2 is molded integrally with a pair of the support portions 3, that is, a front support portion 3A and a back support portion 3B. A soft material can be adopted as the material of the intraocular lens 1. Various soft resin materials such as a single component, for example, butyl acrylate (BA) or hydroxyethylmethacrylate (HEMA), or a composite material of acrylate ester and methacrylate ester can be adopted as the soft material. In the embodiment, what is called the one-piece intraocular lens 1 is illustrated by example. In terms of this, at least a part of the technology illustrated by example in the present disclosure can also be applied to what is called a three-piece intraocular lens having the optical portion 2 and the support portion 3 that are formed separately.

The optical portion 2 gives a predetermined refractive power to a patient's eye. The optical portion 2 has a disc shape. An optical axis L of the optical portion 2 extends in the up-and-down direction through the center of the optical portion 2. As described below, the optical portion 2 includes a first surface 2A that comes into contact with the top portion 132 of the placement portion 130, and a second surface 2B formed on the opposite side to the first surface 2A, as end surfaces in the optical axis L direction. The support portions 3 support the optical portion 2 in the eye. As an example, the intraocular lens 1 of the embodiment is provided with the front support portion 3A and the back support portion 3B as the pair of the support portions 3. Moreover, the front support portion 3A and the back support portion 3B curve and extend outward in the radial direction from an outer edge portion 2C of the optical portion 2. The front support portion 3A and the back support portion 3B are formed at positions that are symmetric about a point with respect to the optical axis L being the center of the optical portion 2.

The front support portion 3A includes a basal portion 6A that is connected to the outer edge portion 2C of the optical portion 2 via a connection portion 4A. Furthermore, the front support portion 3A has a loop shape that curves in the circumferential direction and includes an open distal end portion 8A (in other words, the distal end portion 8A is structured so as to be a free end). The front support portion 3A extends outward in the radial direction from the outer edge portion 2C of the optical portion 2, and is located on the front side in the axial direction, that is, a side closer to the insertion portion 182, in the tapered portion 189 as described below.

The back support portion 3B includes a basal portion 6B that is connected to the outer edge portion 2C of the optical portion 2 via a connection portion 4B. Furthermore, the back support portion 3B has a loop shape that curves in the circumferential direction and includes an open distal end portion 8B (in other words, the distal end portion 8B is structured so as to be a free end). The back support portion 3B extends outward in the radial direction from the outer edge portion 2C of the optical portion 2, and is located on the back side in the axial direction, which is opposite to the insertion portion 182, in the tapered portion 189 as described below.

The front support portion 3A is located closer to the inclined opening end surface 183 than the optical portion 2 is, in the main body portion 100. The back support portion 3B is located backward of the optical portion 2 (on a side away from the inclined opening end surface 183) in the main body portion 100.

### <Thick-walled Structure of Insertion Portion 182>

In the intraocular lens insertion instrument 10, the insertion portion 182 has a narrow shape. Therefore, the insertion portion 182 has a stress concentrated area where stress that is generated when the folded intraocular lens 1 passes through the insertion portion 182 is concentrated. A load is likely to be applied to the stress concentrated area. The stress conceivable is stress on the folded intraocular lens 1, and stress in response to a repulsive force generated in a direction where the folded intraocular lens 1 recovers an original shape thereof. Therefore, at least a part of the insertion portion 182 has a thick-walled structure to improve resistance to the load to be applied when the intraocular lens 1 is ejected. In the thick-walled structure, the thickness of the stress concentrated area of the insertion portion 182 is set to be relatively greater than the thickness of an area of the insertion portion 182 outside the stress concentrated area as viewed in a direction of the central axis (the extrusion axis A) (in other words, as viewed in a direction along the direction of the central axis of the insertion portion 182 (the extrusion axis A). In the thick-walled structure, the insertion portion 182 may be locally thick in the stress concentrated area. Alternatively, the thickness increases gradually from the area outside the stress concentrated area toward the stress concentrated area.

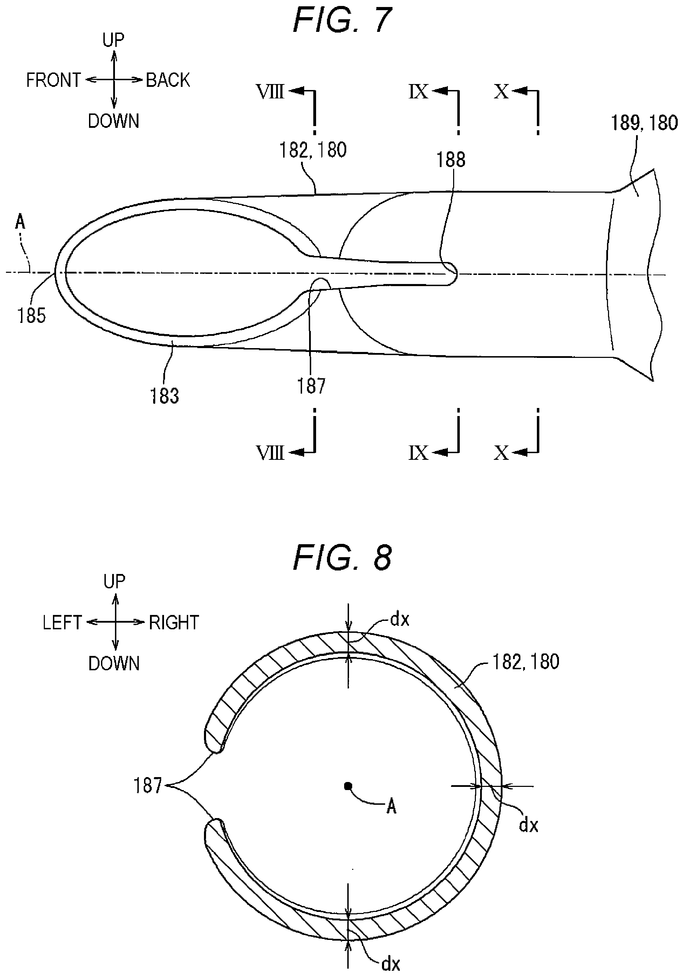

The inclined opening end surface 183 of the insertion portion 182 does not have the thick-walled structure but has a uniform thickness structure. This is because the above-mentioned stress that is generated when the intraocular lens 1 passes through the insertion portion 182 reduces gradually toward the inclined opening end surface 183 since the folded intraocular lens 1 starts being unfolded into an original (unfolded) attitude thereof when being sent out from the inclined opening end surface 183. Fig. 8 is a cross-sectional view taken along line VIII-VIII of Fig. 7. As illustrated in Fig. 8, the thickness (a cross section as viewed from the central axis (the extrusion axis A)) of the inclined opening end surface 183 of the insertion portion 182 is set at a uniform thickness dx. In this manner, each cross section of the inclined opening end surface 183 of the insertion portion 182 is formed in such a manner as to have the same thickness.

A place of the insertion portion 182 where the thick-walled structure is formed is described. The thick-walled structure of the insertion portion 182 is formed on each of a "side surface of a portion that serves as the opening tip portion 185" and a "side surface of a portion that serves as the slit 187" in cross section as viewed in the direction of the central axis (the extrusion axis A) (in other words, in cross section as viewed in the direction along the direction of the central axis of the insertion portion 182 (the extrusion axis A). Furthermore, the thick-walled structures of the above-mentioned side surfaces are formed continuously with the part backward of the inclined opening end surface 183 (that is, the cylindrical portion of the insertion portion 182). In other words, the stress concentrated area is generated in an area on an inner surface of the cylinder backward of the inclined opening end surface 183, on the inner surface of the insertion portion 182.

In terms of the volume of the bore of the insertion portion 182, the filling rate of the folded intraocular lens 1 in the insertion portion 182 is 150% to 200%. Hence, the pressure inside the insertion portion 182 increases accompanied by the insertion of the intraocular lens 1. The intraocular lens 1 tends to increase the thickness at the center of the optical portion 2 with increasing diopter power. Therefore, particularly if the intraocular lens 1 has high diopter power (is thick), the pressure inside the insertion portion 182 tends to increase. Due to the internal pressure, when the intraocular lens 1 passes through the insertion portion 182, the stress concentrated area is generated in the area on the inner surface of the cylinder backward of the inclined opening end surface 183, on the inner surface of the insertion portion 182.

Moreover, there is also a form in which stress is concentrated in response to a repulsive force generated in the direction where the folded intraocular lens 1 recovers the original shape. The vicinity of the basal portion 6B of the back support portion 3B of the intraocular lens 1 that is pushed forward in the axial direction by the pushing member 310 tends to be pressed further against the inner surface of the insertion portion 182 as the back support portion 3B is folded. Hence, the repulsive force generated in the recovering direction is likely to partially increase in the back support portion. The back support portion 3B of the folded intraocular lens 1 passe the vicinity of the "side surface of the portion that serves as the slit 187." Hence, the thick-walled structure is formed in the area.

Moreover, a pushing force acts from the distal end of the pushing member 310 toward the opposite direction (for example, to the right) where the basal portion 6B of the back support portion 3B is not placed, due to the repulsive force generated in the direction where the back support portion 3B recovers an original shape thereof. Hence, a load is likely to be partially applied to the inner surface on the opposite side of the insertion portion 182 where the back support portion 3B is not placed. Therefore, the thick-walled structure is formed in the area of the "side surface of the portion that serves as the opening tip portion 185", which is located at a position that is symmetric about a point with respect to the "side surface of the portion that serves as the slit 187."

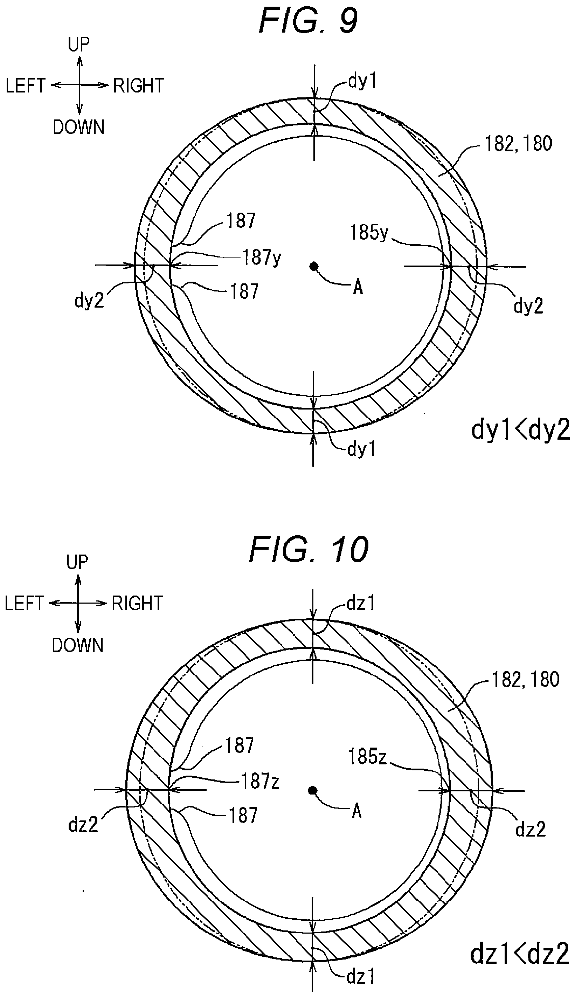

The stress concentrated areas generated in the insertion portion 182 are the areas of the cylinder backward of the inclined opening end surface 183, and are the "side surface of the portion that serves as the opening tip portion 185 (185y of Fig.9 and 185z of Fig. 10)" and the "side surface of the portion that serves as the slit 187 (187y of Fig. 9 and 187z of Fig. 10)."

Fig. 9 is a cross-sectional view taken along line IX-IX of Fig. 7. A cross section illustrated in Fig. 9 is a cross section at a position of a slit root 188 of the slit 187. The insertion portion 182 includes the stress concentrated areas 185y and 187y in the cross section. The stress concentrated areas 185y and 187y are areas where the stress is concentrated in response to the repulsive force generated in the direction where the folded intraocular lens 1 recovers the original shape. The stress concentrated areas 185y and 187y are formed at positions that are symmetric about a point with respect to the extrusion axis A (the central axis). The stress concentrated area 185y is formed on the side surface of the portion, which serves as the opening tip portion 185, of the insertion portion 182. The stress concentrated area 187y is formed on the side surface of the portion, which serves as the slit 187, of the insertion portion 182. Both of the stress concentrated areas 185y and 187y have a thickness dy2. On the other hand, a thickness dy1 of areas of the insertion portion 182 outside the stress concentrated areas 185y and 187y is set to be less than the thickness dy2 (dy1 < dy2). The thickness dy2 of the stress concentrated areas 185y and 187y is the maximum thickness of the cross section. The thickness of the insertion portion 182 changes in such a manner as to gradually reduce from the thickness dy2 to the thickness dy1. In other words, the side surface of the insertion portion 182 is continuous in such a manner as to change in thickness from the thickness dy2 to the thickness dy1 and to have smooth inner and outer shapes in the cross section illustrated in Fig. 9. Moreover, the inner surface of the cylinder of the insertion portion 182 is circular in cross section. Therefore, extra thickness is added to the radially outer sides in the stress concentrated areas 185y and 187y.

Fig. 10 is a cross-sectional view taken along line X-X of Fig. 7. A cross section illustrated in Fig. 10 is a cross section at a position backward of the slit root 188 of the slit 187. The insertion portion 182 includes the stress concentrated areas 185z and 187z in the cross section. The stress concentrated areas 185z and 187z are areas where the stress is concentrated in response to the repulsive force generated in the direction where the folded intraocular lens 1 recovers the original shape. The stress concentrated areas 185z and 187z are formed at positions that are symmetric about a point with respect to the extrusion axis A (the central axis). The stress concentrated area 185z is formed on the side surface of the portion, which serves as the opening tip portion 185, of the insertion portion 182. The stress concentrated area 187z is formed on the side surface of the portion, which serves as the slit 187, of the insertion portion 182. Both of the stress concentrated areas 185z and 187z have a thickness dz2. On the other hand, a thickness dz1 of areas of the insertion portion 182 outside the stress concentrated areas 185z and 187z is set to be less than the thickness dz2 (dz1 < dz2). The thickness dz2 of the stress concentrated areas 185z and 187z is the maximum thickness of the cross section. The thickness of the insertion portion 182 changes in such a manner as to gradually reduce from the thickness dz2 to the thickness dz1. In other words, the side surface of the insertion portion 182 is continuous in such a manner as to change in thickness from the thickness dz2 to the thickness dz1 and to have smooth inner and outer shapes, in the cross section illustrated in Fig. 10. Moreover, the inner surface of the cylinder of the insertion portion 182 is circular in cross section. Therefore, extra thickness is added to the radially outer sides in the stress concentrated areas 185z and 187z.

The thick-walled structure of the insertion portion 182 may be formed on at least one of the "side surface of the portion that serves as the opening tip portion 185" and the "side surface of the portion that serves as the slit 187." Moreover, the thick-walled structure of the insertion portion 182 may be formed on a part of the insertion portion 182 backward of the inclined opening end surface 183. Moreover, both of the stress concentrated areas 185y and 187y may be formed with the same thickness dy2 as described above, or may be formed with different thicknesses. Moreover, the stress concentrated areas 185z and 187z may be formed with the same thickness dz2 as described above, or may be formed with different thicknesses.

Operations and effects based on the adoption of the technology illustrated by example in the embodiment are described with reference to Figs. 11 to 18. Firstly, an operator moves the intraocular lens 1, which is held in the placement portion 130, to a standby position where the plunger 300 can push the intraocular lens 1.

As illustrated in Fig. 11, the basal portion 6A of the front support portion 3A of the intraocular lens 1, which is placed in the placement portion 130, is located on the right side (on the upper side in Fig. 11) relative to the extrusion axis A, and the basal portion 6B of the back support portion 3B is located on the left side (on the lower side in Fig. 11) relative to the extrusion axis A. The intraocular lens insertion instrument 10 of the embodiment is what is called a preset instrument that is preloaded with the intraocular lens 1. However, the technology illustrated by example in the present disclosure can also be applied to, for example, an intraocular lens insertion instrument of a type that is loaded with the intraocular lens 1 immediately before the intraocular lens 1 is inserted into the patient's eye.

The operator injects a filler (for example, a viscoelastic substance or water) into the placement portion 130 by means of, for example, a syringe, and then starts moving the plunger 300 forward. As a result, as illustrated in Fig. 11, the pushing member 310 of the plunger 300 comes into contact with a part of the back support portion 3B of the intraocular lens 1.

As illustrated in Fig. 12, when the plunger 300 is pressed further forward, the back support portion 3B is moved by the pushing member 310 in a direction closer to the optical portion 2 (that is, forward). The back support portion 3B then moves onto the first surface 2A of the optical portion 2 (the side pointing out of the page in Fig. 12) while deforming, and is folded onto the surface. The distal end portion 8B of the back support portion 3B faces forward. In this manner, the back support portion 3B is tucked.

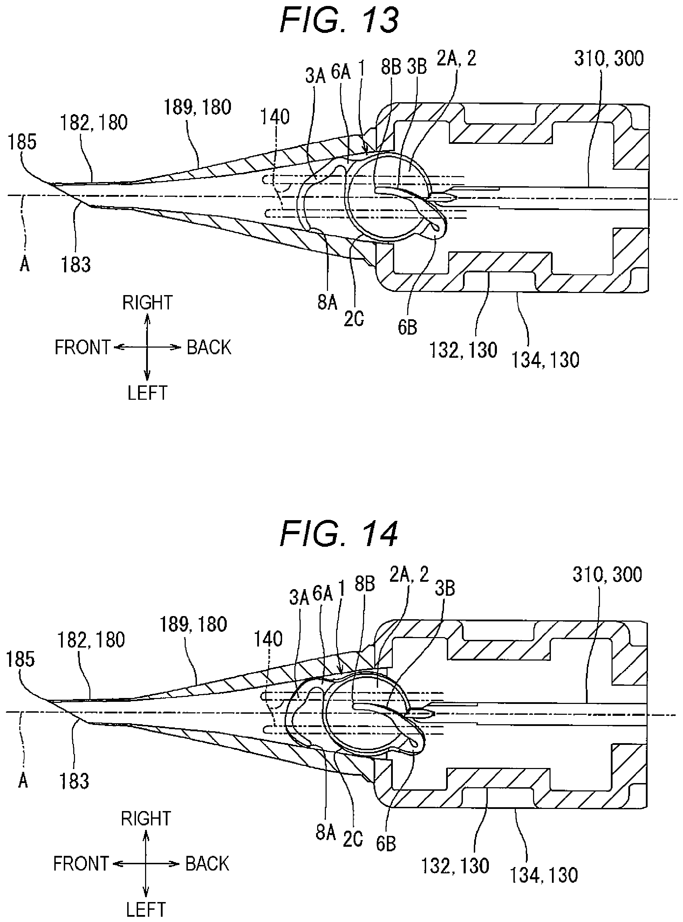

As illustrated in Fig. 13, when the plunger 300 is pressed further forward, the intraocular lens 1 reaches the nozzle 180 and advances toward an inner wall of the tapered portion 189 that becomes gradually thinner at the distal end. The front support portion 3A is located forward of, that is, on a side closer to the insertion portion 182 than, the optical portion 2 in the axial direction in the tapered portion 189. The back support portion 3B is located backward of the optical portion 2, that is, on a side opposite to the insertion portion 182, in the axial direction.

As illustrated in Fig. 14, the plunger 300 is pressed further forward. The optical portion 2 of the intraocular lenses 1 starts being deformed into a roll along the inner wall of the tapered portion 189 since the inner wall has a curved surface.

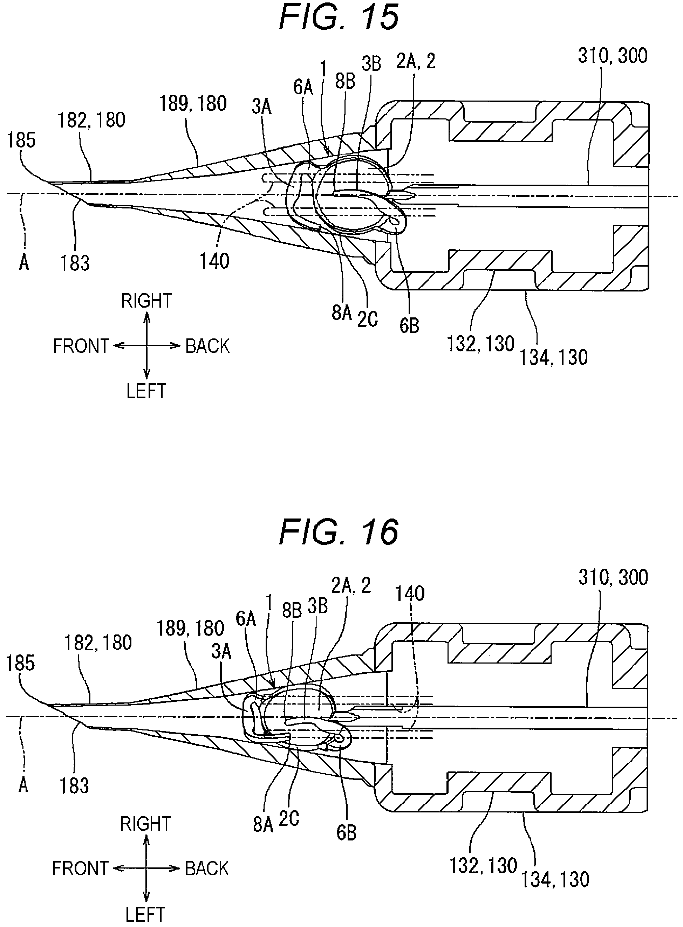

As illustrated in Fig. 15, when the plunger 300 is pressed further forward, the intraocular lens 1 is deformed further into a roll along an inner wall of the nozzle 180.

As illustrated in Fig. 16, when the plunger 300 is pressed further forward, the intraocular lens 1 is deformed further into a roll along the inner wall of the nozzle 180. Moreover, the front support portion 3A becomes caught on the inner wall of the tapered portion 189, and moves closer to the optical portion 2 pushed by the pushing member 310. The front support portion 3A then moves onto the first surface 2A of the optical portion 2 (the side pointing out of the page in Fig. 16) while deforming, and is folded onto the surface. The distal end portion 8A of the front support portion 3A faces backward. In this manner, the front support portion 3A is tucked.

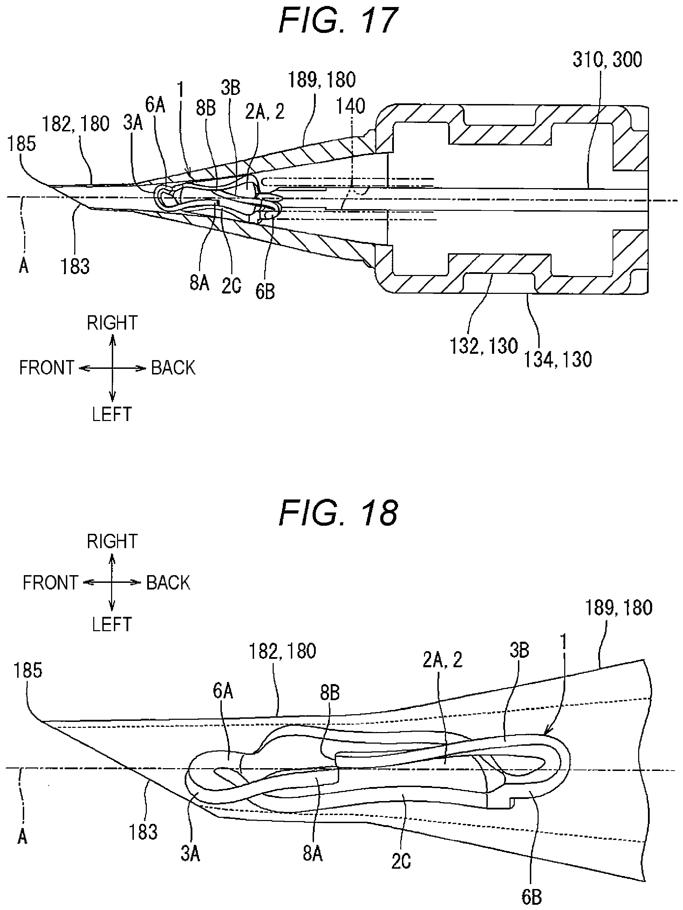

As illustrated in Fig. 17, when the plunger 300 is pressed further forward, the intraocular lens 1 is folded into a reduced size with the front support portion 3A and the back support portion 3B tucked.

As illustrated in Figs. 17 and 18, as the intraocular lens 1 is pushed forward in the axial direction by the pushing member 310 in the tapered portion 189, the intraocular lens 1 becomes folded in such a manner that, adjusting to the passage shape of the tapered portion 189, the distal end portion 8A of the front support portion 3A and the distal end portion 8B of the back support portion 3B are folded onto the optical portion 2 in a direction of moving closer to each other and the optical portion 2 wrap the front support portion 3A and the back support portion 3B.

The back support portion 3B of the intraocular lens 1 that is pushed forward in the axial direction by the pushing member 310 has the tendency that as it is folded, the vicinity of the basal portion 6B of the back support portion 3B is pressed against the inner surface of the insertion portion 182. Hence, the repulsive force generated in the recovering direction in the back support portion 3B is likely to partially increase on the inner surface. The back support portion 3B of the folded intraocular lens 1 passes the vicinity of the "side surface of the portion that serves as the slit 187 (187y of Fig. 9 and 187z of Fig. 10)." Hence, the thick-walled structure is formed on each of these areas. In other words, the stress concentrated areas 187y and 187z are the areas where the stress is concentrated in response to the repulsive force generated in the direction where the back support portion 3B of the folded intraocular lens 1 recovers the original shape.

Moreover, due to the repulsive force generated in the direction where the back support portion 3B recovers the original shape, the pushing force acts from the distal end of the pushing member 310 toward the opposite direction where the basal portion 6B of the back support portion 3B is not placed. Hence, a load is likely to be partially applied to the inner surface on the opposite side of the insertion portion 182 where the back support portion 3B is not placed. Therefore, the thick-walled structure is formed in each of the areas of the "side surface of the portion that serves as the opening tip portion 185 (185y of Fig. 9 and 185z of Fig. 10)" that is located at the position that is symmetric about a point with respect to the "side surface of the portion that serves as the slit 187."

The stress concentrated areas generated on the insertion portion 182 are the areas of the cylinder backward of the inclined opening end surface 183, and are the "side surface of the portion that serves as the opening tip portion 185 (185y of Fig. 9 and 185z of Fig. 10)," and the "side surface of the portion that serves as the slit 187 (187y of Fig. 9 and 187z of Fig. 10)." The thicknesses of the areas are set at the thicknesses dy2 and dz2. Consequently, these areas are made thicker than the areas having the thicknesses dy1 and dz1 outside the stress concentrated areas (dz1 <dz2). Therefore, the thick-walled structure allows efficiently improving resistance to a load on the insertion portion 182 in the nozzle end to be applied when the intralocular lens 1 is ejected.

The intraocular lens 1 is then inserted into the eye from the inclined opening end surface 183. When the insertion portion 182 of the intraocular lens insertion instrument 10 is inserted into the bag of the crystalline lens, the insertion portion 182 is inserted with the opening of the inclined opening end surface 183 facing the posterior capsule of the crystalline lens. The intraocular lens 1 is sent out from the inclined opening end surface 183 with the following behavior. Immediately before the intraocular lens 1 is sent out from the inclined opening end surface 183, the tucked back support portion 3B becomes sandwiched between the pushing member 310 and the inner wall of the nozzle 180 and starts being unfolded into an original (unfolded) attitude thereof while the optical portion 2 rotates about the extrusion axis A. Lastly, when the back support portion 3B is sent out from the inclined opening end surface 183, the intraocular lens 1 is in an attitude that the first surface 2A faces the anterior capsule and the second surface 2B faces the posterior capsule, and is placed within the crystalline lens.

In this manner, the intraocular lens insertion instrument according to the embodiment of the present disclosure can improve resistance to a load on the insertion portion 182 in the nozzle end to be applied when the intraocular lens 1 is ejected.

Moreover, it is possible to improve resistance to loads on the areas of the insertion portion 182 in the nozzle end where the stress is concentrated in response to the repulsive force generated in the direction where the folded intraocular lens 1 recovers the original shape (the force in the direction where the intraocular lens 1 recovers the original (unfolded) attitude).

Moreover, the back support portion 3B of the intraocular lens 1 that is pushed forward in the axial direction by the pushing member 310 has the tendency that as it is folded, the vicinity of the basal portion 6B of the back support portion 3B is pressed against the inner surface of the insertion portion 182. Hence, the repulsive force generated in the recovering direction is likely to increase in the back support portion. Moreover, the pushing force acts from the distal end of the pushing member 310 toward the opposite direction where the basal portion 6B of the back support portion 3B is not placed, due to the repulsive force generated in the direction where the back support portion 3B recovers the original shape. Hence, a load is likely to be applied to the inner surface on the opposite side of the insertion portion 182 where the back support portion 3B is not placed. Therefore, the thick-walled structure is provided to each of the stress concentrated areas 185y, 185z, 187y, and 187z; therefore, it is possible to efficiently improve resistance to a load on the insertion portion 182 in the nozzle end to be applied when the intraocular lens 1 is ejected.

Moreover, the stress concentrated areas 185y, 185z, 187y, and 187z are on the above-mentioned side surface of the portion, which serves as the opening tip portion 185, of the insertion portion 182, or the side surface of the portion, which serves as the slit 187, of the insertion portion 182. Hence, the thick-walled structure is provided to at least one of the areas; therefore, it is possible to selectively improve resistance to a load on the insertion portion 182 in the nozzle end to be applied when the intraocular lens 1 is ejected.

Moreover, the thick-walled structure is provided to each of the side surface of the portion, which serves as the opening tip portion 185, of the insertion portion 182, and the side surface of the portion, which serves as the slit 187, of the insertion portion 182. Consequently, it is possible to further improve resistance to a load on the insertion portion 182 in the nozzle end to be applied when the intraocular lens 1 is ejected.

Moreover, the thick-walled structure may be formed in such a manner that the side surface of the portion, which serves as the opening tip portion 185, of the insertion portion 182 is different in thickness from the side surface of the portion, which serves as the slit 187, of the insertion portion 182. In other words, the area of the side surface of the portion, which serves as the opening tip portion 185, of the insertion portion 182, or the side surface of the portion, which serves as the slit 187, of the insertion portion 182, to whichever a heavier load is applied, is made thicker; therefore, it is possible to excellently improve resistance to a load on the insertion portion 182.

Moreover, the thick-walled structure may be formed in such a manner that the side surface of the portion, which serves as the opening tip portion 185, of the insertion portion 182 is equal in thickness to the side surface of the portion, which serves as the slit 187, of the insertion portion 182. In other words, the side surface of the portion, which serves as the opening tip portion 185, of the insertion portion 182, and the side surface of the portion, which serves as the slit 187, of the insertion portion 182 are formed in such a manner as to have the same thickness; therefore, it is possible to prevent the areas where the stress is concentrated from being distributed unevenly to one side.

Up to this point, the embodiment of the present disclosure has been described. However, the intraocular lens insertion instrument of the present disclosure is not limited to the above embodiment, and can be carried out in various other forms. For example, in the above-mentioned embodiment, the thick-walled structure is formed in such a manner as to include the same resin material. In terms of this, the thick-walled structure may be formed in such a manner that the thicker portion includes a higher-strength resin (for example, two-color molding). Moreover, the thicker portion may be formed in such a manner as to include another higher-strength material.

Moreover, the intraocular lens insertion instrument according to the embodiment of the present disclosure may be the following first intraocular lens insertion instrument. The first intraocular lens insertion instrument is an intraocular lens insertion instrument that pushes an intraocular lens placed in a tubular main body portion from the back toward the front of the main body portion in an axial direction by means of a rod-like pushing member inserted into the main body portion, and pushes the intraocular lens in an folded state thereof from a distal end of the main body portion to the outside, thereby inserting the intraocular lens into the eye, the intraocular lens insertion instrument including a cylindrical insertion portion placed in the distal end of the main body portion to be inserted into the eye, in which the insertion portion includes a stress concentrated area where stress to be generated upon the folded intraocular lens passing through the insertion portion is concentrated, and a thick-walled structure where the thickness of the stress concentrated area of the insertion portion is set to be relatively greater than the thickness of an area outside the stress concentrated area as the insertion portion is viewed in a direction of a central axis thereof is formed on at least a part of the insertion portion.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

An intraocular lens insertion instrument includes: a tubular main body portion where an intraocular lens is placed; a rod-like pushing member inserted into the main body portion and configured to press the intraocular lens from a back toward a front of the main body portion in an axial direction, thereby push the intraocular lens in a folded state from a distal end of the main body portion to the outside, and then insert the intraocular lens into an eye; and a cylindrical insertion portion placed in the distal end of the main body portion to be inserted into the eye, in which the insertion portion includes a stress concentrated area where stress to be generated upon the folded intraocular lens passing through the insertion portion is concentrated, and the insertion portion includes a thick-walled structure in at least a part thereof, in which a thickness of the stress concentrated area of the insertion portion is set to be relatively greater than a thickness of an area of the insertion portion outside the stress concentrated area as the insertion portion is viewed in a direction of a central axis thereof.

## Claims

1. An intraocular lens insertion instrument comprising:
a tubular main body portion (100) where an intraocular lens (1) is placed;
a rod-like pushing member (310) inserted into the main body portion (100) and configured to press the intraocular lens (1) from a back toward a front of the main body portion (100) in an axial direction, thereby push the intraocular lens (1) in a folded state from a distal end of the main body portion (100) to the outside, and then insert the intraocular lens (1) into an eye; and
a cylindrical insertion portion (182) placed in the distal end of the main body portion (100) to be inserted into the eye, wherein
the insertion portion (182) includes a stress concentrated area (185y, 185z, 187y, 187z) where stress to be generated upon the folded intraocular lens (1) passing through the insertion portion (182) is concentrated,
the insertion portion (182) includes, in a distal end thereof, an inclined opening end surface (183) inclined relative to a virtual plane orthogonal to the central axis of the insertion portion (182), and
the insertion portion (182) includes:
an opening tip portion (185) located at an extreme tip of the inclined opening end surface (183); and
a slit (187) cut backward in the axial direction from an end surface located at a backmost end of the inclined opening end surface (183), **characterized in that**
the insertion portion (182) includes a thick-walled structure in at least a part thereof, in which a thickness of the stress concentrated area (185y, 185z, 187y, 187z) of the insertion portion (182) is set to be relatively greater than a thickness of an area of the insertion portion (182) outside the stress concentrated area (185y, 185z, 187y, 187z) as the insertion portion (182) is viewed in a direction of a central axis thereof, and
the thick-walled structure is formed on at least one of a side surface of a portion, which serves as the opening tip portion (185), of the insertion portion (182), and a side surface of a portion, which serves as the slit (187), of the insertion portion (182).

2. The intraocular lens insertion instrument according to claim 1, wherein the stress concentrated area (185y, 185z, 187y, 187z) of the insertion portion (182) is an area where the stress is concentrated in response to a repulsive force generated in a direction where the folded intraocular lens (1) recovers an original shape thereof.

3. The intraocular lens insertion instrument according to claim 1 or 2, further comprising a tapered portion (189) continuous with a back of the insertion portion (182) in the axial direction, the tapered portion (189) having a hollow passage shape where a passage through which the intraocular lens (1) passes gradually reduces in area toward a distal end thereof, wherein
the intraocular lens (1) includes:
a disc-shaped optical portion (2);
a front support portion (3A) extending outward in a radial direction from an outer edge portion (2C) of the optical portion (2), the front support portion (3A) being located on a front side in the axial direction, which is a side closer to the insertion portion (182), in the tapered portion (189); and
a back support portion (3B) extending outward in the radial direction from the outer edge portion (2C) of the optical portion (2), the back support portion (3B) being located on a back side in the axial direction, which is opposite to the insertion portion (182), in the tapered portion (189),
as the intraocular lens (1) is pushed forward in the axial direction by the pushing member (310) in the tapered portion (189), the intraocular lens (1) becomes folded in such a manner that, adjusting to the passage shape of the tapered portion (189), a distal end portion of the front support portion (3A) and a distal end portion of the back support portion (3B) are folded onto the optical portion (2) in a direction of moving closer to each other and the optical portion (2) wrap the front support portion (3A) and the back support portion (3B), and
the stress concentrated area (185y, 185z, 187y, 187z) is an area where the stress is concentrated in response to a repulsive force generated in a direction where the back support portion (3B) of the folded intraocular lens (1) recovers the original shape.

4. The intraocular lens insertion instrument according to any one of claims 1 to 3, wherein the thick-walled structure is formed on each of the side surface of the portion, which serves as the opening tip portion (185), of the insertion portion (182), and the side surface of the portion, which serves as the slit (187), of the insertion portion (182).

5. The intraocular lens insertion instrument according to claim 4, wherein the thick-walled structure is formed in such a manner that the side surface of the portion, which serves as the opening tip portion (185), of the insertion portion (182) is different in thickness from the side surface of the portion, which serves as the slit (187), of the insertion portion (182).

6. The intraocular lens insertion instrument according to claim 4, wherein the thick-walled structure is formed in such a manner that the side surface of the portion, which serves as the opening tip portion (185), of the insertion portion (182) is equal in thickness to the side surface of the portion, which serves as the slit (187), of the insertion portion (182).

## Patentansprüche

1. Instrument zum Einsetzen einer Intraokularlinse, umfassend:
einen röhrenförmigen Hauptkörperabschnitt (100), in dem eine Intraokularlinse (1) platziert ist;
ein stabförmiges Schiebeelement (310), das in den Hauptkörperabschnitt (100) eingesetzt ist und so konfiguriert ist, dass es die Intraokularlinse (1) von einer Rückseite zu einer Vorderseite des Hauptkörperabschnitts (100) in einer axialen Richtung drückt, wodurch die Intraokularlinse (1) in einem gefalteten Zustand von einem distalen Ende des Hauptkörperabschnitts (100) nach außen geschoben wird, und dann die Intraokularlinse (1) in ein Auge einsetzt; und
einen zylindrischen Einführabschnitt (182), der in dem distalen Ende des Hauptkörperabschnitts (100) platziert ist, um in das Auge eingeführt zu werden, wobei
der Einführabschnitt (182) einen spannungskonzentrierten Bereich (185y, 185z, 187y, 187z) aufweist, in dem eine Spannung konzentriert ist, die auf die gefaltete Intraokularlinse (1), die durch den Einführabschnitt (182) hindurchgeht, erzeugt wird,
der Einführabschnitt (182) an seinem distalen Ende eine geneigte Öffnungsendfläche (183) aufweist, die relativ zu einer virtuellen Ebene orthogonal zur Mittelachse des Einführabschnitts (182) geneigt ist, und
der Einführabschnitt (182) aufweist:
einen Öffnungsspitzenabschnitt (185), der sich an einer äußersten Spitze der geneigten Öffnungsendfläche (183) befindet; und
einen Schlitz (187), der von einer Endfläche, die sich an einem hintersten Ende der geneigten Öffnungsendfläche (183) befindet, in der axialen Richtung nach hinten geschnitten ist, **dadurch gekennzeichnet, dass**
der Einführabschnitt (182) eine dickwandige Struktur in mindestens einem Teil davon aufweist, in dem eine Dicke des spannungskonzentrierten Bereichs (185y, 185z, 187y, 187z) des Einführabschnitts (182) so eingestellt ist, dass sie relativ größer als eine Dicke eines Bereichs des Einführabschnitts (182) außerhalb des spannungskonzentrierten Bereichs (185y, 185z, 187y, 187z) ist, wenn der Einführabschnitt (182) in einer Richtung einer Mittelachse davon betrachtet wird, und
die dickwandige Struktur auf mindestens einer Seitenfläche eines Abschnitts, der als der Öffnungsspitzenabschnitt (185) dient, des Einführabschnitts (182), und einer Seitenfläche eines Abschnitts, der als der Schlitz (187) dient, des Einführabschnitts (182) ausgebildet ist.

2. Instrument zum Einsetzen einer Intraokularlinse nach Anspruch 1, wobei der spannungskonzentrierte Bereich (185y, 185z, 187y, 187z) des Einführabschnitts (182) ein Bereich ist, in dem die Spannung als Reaktion auf eine Abstoßungskraft konzentriert ist, die in einer Richtung erzeugt wird, in der die gefaltete Intraokularlinse (1) ihre ursprüngliche Form wiedererlangt.

3. Instrument zum Einsetzen einer Intraokularlinse nach Anspruch 1 oder 2, ferner umfassend einen sich in der axialen Richtung an eine Rückseite des Einführabschnitts (182) anschließenden verjüngenden Abschnitt (189), wobei der sich verjüngende Abschnitt (189) die Form eines hohlen Durchgangs aufweist, in dem sich die Fläche eines Durchgangs, durch den die Intraokularlinse (1) hindurchgeht, in Richtung seines distalen Endes allmählich verringert, wobei
die Intraokularlinse (1) aufweist:
einen scheibenförmigen optischen Teil (2);
einen vorderen Stützabschnitt (3A), der sich in einer radialen Richtung von einem äußeren Kantenabschnitt (2C) des optischen Abschnitts (2) nach außen erstreckt, wobei der vordere Stützabschnitt (3A) auf einer Vorderseite in der axialen Richtung, die eine Seite ist, die näher an dem Einführabschnitt (182) liegt, in dem sich verjüngenden Abschnitt (189) angeordnet ist; und
einen hinteren Stützabschnitt (3B), der sich in der radialen Richtung von dem äußeren Kantenabschnitt (2C) des optischen Abschnitts (2) nach außen erstreckt, wobei der hintere Stützabschnitt (3B) auf einer Rückseite in der axialen Richtung, die dem Einführabschnitt (182) gegenüberliegt, in dem sich verjüngenden Abschnitt (189) angeordnet ist,
wenn die Intraokularlinse (1) durch das Schiebeelement (310) in dem sich verjüngenden Abschnitt (189) in der axialen Richtung nach vorne geschoben wird, die Intraokularlinse (1) so gefaltet wird, dass unter Anpassung an die Durchgangsform des sich verjüngenden Abschnitts (189) ein distaler Endabschnitt des vorderen Stützabschnitts (3A) und ein distaler Endabschnitt des hinteren Stützabschnitts (3B) auf den optischen Abschnitt (2) in einer Richtung der Annäherung aneinander gefaltet werden und der optische Abschnitt (2) den vorderen Stützabschnitt (3A) und den hinteren Stützabschnitt (3B) umhüllt, und
der spannungskonzentrierte Bereich (185y, 185z, 187y, 187z) ein Bereich ist, in dem die Spannung als Reaktion auf eine Abstoßungskraft konzentriert ist, die in einer Richtung erzeugt wird, in der der hintere Stützabschnitt (3B) der gefalteten Intraokularlinse (1) die ursprüngliche Form wiedererlangt.

4. Instrument zum Einsetzen einer Intraokularlinse nach einem der Ansprüche 1 bis 3, wobei die dickwandige Struktur sowohl auf der Seitenfläche des Abschnitts, der als Öffnungsspitzenabschnitt (185) dient, des Einführabschnitts (182) als auch auf der Seitenfläche des Abschnitts, der als Schlitz (187) dient, des Einführabschnitts (182) ausgebildet ist.

5. Instrument zum Einsetzen einer Intraokularlinse nach Anspruch 4, wobei die dickwandige Struktur so ausgebildet ist, dass die Seitenfläche des Abschnitts, der als Öffnungsspitzenabschnitt (185) dient, des Einführabschnitts (182) eine andere Dicke aufweist als die Seitenfläche des Abschnitts, der als Schlitz (187) dient, des Einführabschnitts (182).

6. Instrument zum Einsetzen einer Intraokularlinse nach Anspruch 4, wobei die dickwandige Struktur so ausgebildet ist, dass die Seitenfläche des Abschnitts, der als Öffnungsspitzenabschnitt (185) dient, des Einführabschnitts (182) die gleiche Dicke aufweist wie die Seitenfläche des Abschnitts, der als Schlitz (187) dient, des Einführabschnitts (182).

## Revendications

1. Instrument d'insertion de lentille intraoculaire comprenant :
une partie de corps principal tubulaire (100) dans laquelle est placée une lentille intraoculaire (1) ;
un élément de poussée en forme de tige (310) inséré dans la partie de corps principal (100) et configuré pour presser la lentille intraoculaire (1) de l'arrière vers l'avant de la partie de corps principal (100) dans une direction axiale, poussant ainsi la lentille intraoculaire (1) à l'état plié d'une extrémité distale de la partie de corps principal (100) vers l'extérieur, puis insérer la lentille intraoculaire (1) dans un œil ; et
une partie d'insertion cylindrique (182) placée à l'extrémité distale de la partie de corps principal (100) pour être insérée dans l'œil, dans laquelle
la partie d'insertion (182) comprend une zone de concentration de contrainte (185y, 185z, 187y, 187z) dans laquelle la contrainte devant être générée lors du passage de la lentille intraoculaire (1) pliée à travers la partie d'insertion (182) se concentre,
la partie d'insertion (182) comprend, dans son extrémité distale, une surface d'extrémité d'ouverture inclinée (183) qui est inclinée par rapport à un plan virtuel orthogonal à l'axe central de la partie d'insertion (182), et
la partie d'insertion (182) comprend :
une partie de pointe d'ouverture (185) située à une pointe extrême de la surface d'extrémité d'ouverture inclinée (183) ; et
une fente (187) coupée vers l'arrière dans la direction axiale à partir d'une surface d'extrémité située à l'extrémité arrière de la surface d'extrémité d'ouverture inclinée (183), **caractérisé en ce que**
la partie d'insertion (182) comprend une structure à parois épaisses dans au moins une partie de celle-ci, dans laquelle une épaisseur de la zone de concentration de contrainte (185y, 185z, 187y, 187z) de la partie d'insertion (182) est définie pour être relativement plus grande qu'une épaisseur d'une zone de la partie d'insertion (182) à l'extérieur de la zone de concentration de contrainte (185y, 185z, 187y, 187z) lorsque la partie d'insertion (182) est vue dans une direction de son axe central, et
la structure à parois épaisses est formée sur au moins une parmi une surface latérale d'une partie, qui sert de partie de pointe d'ouverture (185), de la partie d'insertion (182), et une surface latérale d'une partie, qui sert de fente (187), de la partie d'insertion (182).

2. Instrument d'insertion de lentille intraoculaire selon la revendication 1, dans lequel la zone de concentration de contrainte (185y, 185z, 187y, 187z) de la partie d'insertion (182) est une zone dans laquelle la contrainte est concentrée en réponse à une force de répulsion générée dans une direction dans laquelle la lentille intraoculaire (1) pliée retrouve sa forme d'origine.

3. Instrument d'insertion de lentille intraoculaire selon la revendication 1 ou 2, comprenant en outre une partie effilée (189) continue avec l'arrière de la partie d'insertion (182) dans la direction axiale, la partie effilée (189) ayant une forme de passage creux où un passage à travers lequel la lentille intraoculaire (1) passe réduit graduellement en surface vers son extrémité distale, dans lequel
la lentille intraoculaire (1) comprend :
une partie optique en forme de disque (2) ;
une partie de support avant (3A) s'étendant vers l'extérieur dans une direction radiale à partir d'une partie de bord extérieur (2C) de la partie optique (2), la partie de support avant (3A) étant située sur un côté avant dans la direction axiale, qui est un côté plus proche de la partie d'insertion (182), dans la partie effilée (189) ; et
une partie de support arrière (3B) s'étendant vers l'extérieur dans la direction radiale à partir de la partie de bord extérieur (2C) de la partie optique (2), la partie de support arrière (3B) étant située sur un côté arrière dans la direction axiale, qui est opposée à la partie d'insertion (182), dans la partie effilée (189),
lorsque la lentille intraoculaire (1) est poussée vers l'avant dans la direction axiale par l'élément de poussée (310) dans la partie effilée (189), la lentille intraoculaire (1) est pliée de telle sorte que, en s'adaptant à la forme de passage de la partie effilée (189), une partie d'extrémité distale de la partie de support avant (3A) et une partie d'extrémité distale de la partie de support arrière (3B) sont pliées sur la partie optique (2) dans le sens d'un rapprochement l'une de l'autre et la partie optique (2) enveloppe la partie de support avant (3A) et la partie de support arrière (3B), et
la zone de concentration de contrainte (185y, 185z, 187y, 187z) est une zone dans laquelle la contrainte est concentrée en réponse à une force de répulsion générée dans une direction dans laquelle la partie de support arrière (3B) de la lentille intraoculaire pliée (1) retrouve sa forme d'origine.

4. Instrument d'insertion de lentille intraoculaire selon l'une des revendications 1 à 3, dans lequel la structure à parois épaisses est formée sur chacune de la surface latérale de la partie, qui sert de partie de pointe d'ouverture (185), de la partie d'insertion (182) et de la surface latérale de la partie, qui sert de fente (187), de la partie d'insertion (182).

5. Instrument d'insertion de lentille intraoculaire selon la revendication 4, dans lequel la structure à parois épaisses est formée de telle manière que la surface latérale de la partie, qui sert de partie de pointe d'ouverture (185), de la partie d'insertion (182) est différente en épaisseur de la surface latérale de la partie, qui sert de fente (187), de la partie d'insertion (182).

6. Instrument d'insertion de lentille intraoculaire selon la revendication 4, dans lequel la structure à parois épaisses est formée de telle manière que la surface latérale de la partie, qui sert de partie de pointe d'ouverture (185), de la partie d'insertion (182) est égale en épaisseur à la surface latérale de la partie, qui sert de fente (187), de la partie d'insertion (182).
